# EUROPÄISCHE PATENTSCHRIFT

(11) **EP 4 212 110 B1**
(45) Veröffentlichungstag und Bekanntmachung des Hinweises auf die Patenterteilung: **16.04.2025**
(21) Anmeldenummer: 22152076.0
(22) Anmeldetag: 18.01.2022
(51) Int. Cl.: A61B 17/17

(54) **ZIELVORRICHTUNG**
AIMING DEVICE
DISPOSITIF DE VISÉE

(43) Veröffentlichungstag der Anmeldung: 19.07.2023
(73) Patentinhaber: Createc GmbH & Co. KG, 88046 Friedrichshafen (DE)
(72) Erfinder: Mathys, Dominik, 3066 Stettlen (CH); Vogel, Berthold, 71701 Schwieberdingen (DE); Kopplin, Andris, 78464 Konstanz (DE)
(74) Vertreter: Westphal, Mussgnug & Partner Patentanwälte mbB

(56) Entgegenhaltungen:
- WO-A1-2017/125476
- WO-A1-2018/160445
- DE-U1- 29 804 268
- US-A1- 2012 022 533

## Beschreibung

Die vorliegende Erfindung betrifft eine Zielvorrichtung zum Ausbilden einer Befestigung einer Prothese, insbesondere eines Marknagels mit einem Knochenbefestigungselement an einem Knochen mit den Merkmalen des Patentanspruchs 1.

Derartige Zielvorrichtungen sind aus dem Stand der Technik in unterschiedlichen Ausgestaltungen vorbekannt und dienen der zielgenauen Positionierung eines Knochenbefestigungselements in einer korrespondierenden Ausnehmung einer Prothese, beispielsweise eines Marknagels, um die Prothese fest mit dem Knochen zu verbinden und an diesem zu verankern. Verbreitet werden derartige Zielvorrichtungen um Knochen (z. B. Femur oder Tibia) nach einer Fraktur zu stabilisieren. Beispielsweise ist eine solche Zielvorrichtung aus der US2012 022 533 A1 vorbekannt.

Die verwendeten Prothesen umfassen zu diesem Zweck eine Ausnehmung, durch die das Knochenbefestigungselement eingebracht werden kann, um die Prothese bzw. den Marknagel in dem Knochen zu verankern. In den Knochen kann eine zu der Ausnehmung korrespondiere Ausnehmung durch ein Werkzeug eingebracht werden, wobei die korrespondiere Ausnehmung mittels der Zielvorrichtung mit einer Führungsöffnung passgenau auf die Ausnehmung in der Prothese eingebracht wird. Um bei der Verwendung der Zielvorrichtung beim Einbringen der korrespondierenden Ausnehmung Beschädigungen des umgebenden Gewebes zu verhindern, wird das Gewebe zu dem Werkzeug auf Abstand gehalten.

Neben Gewebespreizern werden auch Schutzhülsen verwendet, die das Werkzeug beim Einbringen der korrespondierenden Ausnehmung in den Knochen abschirmen.

Die vorliegende Erfindung widmet sich der Aufgabe, eine in zweckmäßiger Weise verbesserte Zielvorrichtung vorzuschlagen. Bei der Verwendung einer zuvor beschriebenen Zielvorrichtung sollen Beschädigungen des umgebenden Gewebes beim Einbringen der korrespondierenden Ausnehmung in den Knochen durch das Werkzeug mittels einer Schutzhülse verhindert werden, wobei die Handhabung der Zielvorrichtung einfach und zuverlässig sein soll.

Diese Aufgaben werden durch eine Zielvorrichtung mit den Merkmalen des Patentanspruchs 1 gelöst.

Weitere vorteilhafte Ausgestaltungen der erfindungsgemäßen Zielvorrichtung werden in den Unteransprüchen angegeben.

Die erfindungsgemäße Zielvorrichtung mit den Merkmalen des Patentanspruchs 1 zum Ausbilden einer Befestigung einer Prothese, insbesondere eines Marknagels, an einem Knochen weist ein Haltemittel zum Positionieren der Prothese, insbesondere des Marknagels, in einer ersten Achse, mindestens eine mindestens eine Führungsöffnung bildende Aufnahme, die entlang einer zweiten Achse orientiert ist und die Zielvorrichtung durchbricht und ausgebildet ist, die mindestens eine Schutzhülse aufzunehmen, und mindestens ein Arretierungsmittel auf, welches wenigstens einen elastisch auslenkbaren Finger umfasst, der in Bezug auf die zweite Achse axial und/oder radial ausgelenkt werden kann. Die erste Achse und die zweite Achse schneiden sich, wobei ferner vorgesehen ist, dass der wenigstens eine elastisch auslenkbare Finger in die Führungsöffnung ragt. Der mindestens eine elastisch auslenkbare Finger ist eingerichtet, die Schutzhülse entlang der zweiten Achse festzulegen.

Die vorliegende Erfindung beruht auf der Idee, die Schutzhülse innerhalb der Führungsöffnung durch den wenigstens einen elastisch auslenkbaren Finger festzulegen. Die Schutzhülse muss daher nicht durch medizinisches Personal gehalten werden und läuft weiterhin nicht Gefahr unbeabsichtigt aus der Führungsöffnung herauszufallen.

An dieser Stelle wird angemerkt, dass die Zielvorrichtung mehr als eine eine Führungsöffnung bildende Aufnahme aufweisen kann. Die jeweiligen Führungsöffnungen sind können jeweils entlang einer zweiten Achse orientiert sein, wobei die zweiten Achsen beabstandet zueinander sind. Die zweiten Achsen können zueinander parallel orientiert sein, oder sich in einem gemeinsamen Schnittpunkt schneiden. Der durch die zweiten Achsen eingeschlossene Winkel beträgt vorzugsweise 30° bis 60°.

Der wenigstens eine elastisch auslenkbare Finger kann mit der in die Führungsöffnung eingesetzten Schutzhülse in Wirkkontakt kommen, und eine reibschlüssige Verbindung mit der Schutzhülse bewerkstelligen. Beim Einsetzen der Schutzhülse in die Führungsöffnung wird der wenigstens eine elastische Finger axial und/oder radial ausgelenkt und bringt eine die Schutzhülse festlegende Haltekraft auf.

Durch eine entsprechende Auslegung der Steifigkeit des wenigstens einen elastischen Fingers kann die Haltekraft dimensioniert werden und so die Haltekraft an die jeweilige Anwendung oder an Vorlieben des Anwenders angepasst werden.

Die Führungsöffnung und/oder die Schutzhülse sind bevorzugt zylinderförmig und die Führungsöffnung durchdringt die Zielvorrichtung vollständig. Bevorzugt ist die Führungsöffnung so ausgebildet, dass die Schutzhülse leichtgängig durchgeführt werden kann, beispielsweise mittels einer Spielpassung.

Die Erfindung sieht vor, dass das Arretierungsmittel eine Buchse umfasst. Bevorzugt kann die Buchse ein Hohlzylinder mit einer Durchbrechung sein, wobei die Durchbrechung bevorzugt in der Form und/oder der Abmessung an die Führungsöffnung angepasst oder gleich ist und ferner einen Abschnitt der Führungsöffnung bilden kann.

Die Durchbrechung kann einen polygonen, kreisrunden oder achtförmigen Querschnitt aufweisen, wobei die Durchbrechung derart ausgebildet ist, dass mindestens eine Führungshülse hindurchgeführt werden kann. Der achtförmige Querschnitt kann es ermöglichen, dass die mindestens eine Führungshülse in mindestens zwei Positionierungen parallel und beabstandet zu der zweiten Achse durchgeführt werden kann.

Der wenigstens eine elastische Finger steht von dem Arretierungsmittel bzw. der Buchse radial nach innen oder radial nach innen und axial ab. Die Buchse kann auf beliebige Weise an der Zielvorrichtung befestigt werden, wobei weiter bevorzugt die Durchbrechung und die Führungsöffnung in der zweiten Achse koaxial angeordnet sind.

Gemäß einer weiteren bevorzugten Ausgestaltung kann ds Arretierungsmittel bzw. die Buchse in der Aufnahme der Zielvorrichtung - vorzugsweise vollständig - anordnenbar sein. Insbesondere ist es bevorzugt, wenn die Buchse in der Aufnahme verliersicher anordnenbar ist. Bevorzugt weist die Aufnahme eine Aufweitung nach Art einer Zylinderschraubensenkung auf, in die die Buchse eingesetzt werden kann. Die Buchse wird in der Aufnahme koaxial zu der Führungsöffnung ausgerichtet gehalten.

Darüber hinaus hat es sich als vorteilhaft erwiesen, wenn ein Ringraum in der Aufnahme ausgebildet ist, und dass der mindestens eine Finger in den Ringraum frei beweglich ragen kann. Insbesondere ist es bevorzugt, wenn der Ringraum stirnseitig zu dem Arretierungsmittel bzw. der Buchse ausgebildet ist und einerseits durch die Aufnahme und anderseits durch die Buchse bevorzugt U-förmig umschlossen ist.

Eine bevorzugte Weiterbildung der vorliegenden Erfindung sieht vor, dass in den Ringraum ein Spülkanal mündet. Durch die Spülbohrung kann ein Reinigungsmittel in den Ringraum eingebracht werden, um die Zielvorrichtung nach oder vor der Verwendung zu Reinigen.

Nach Maßgabe einer Weiterbildung mündet der Spülkanal in einer zu der zweiten Achse tangential orientierten Richtung in den Ringraum, wodurch der Ringraum bei der Reinigung effektiv mit einem Reinigungsmittel durchspült werden kann.

Nach Maßgabe einer bevorzugten Weiterbildung der vorliegenden Erfindung ist die Buchse im Querschnitt U-förmig. Die Buchse weist einen inneren Schenkel, einen äußeren Schenkel und einen mittleren Schenkel auf, wobei der mittlere Schenkel den inneren Schenkel und den äußeren Schenkel verbindet. Durch die U-förmige Ausgestaltung der Buchse kann der Ringraum von den Schenkeln der U-förmigen Buchse ausgebildet werden.

Bevorzugt kann ein Verbindungsmittel vorgesehen werden, durch dass das Arretierungsmittel in der Aufnahme kraftschlüssig, formschlüssig und/oder stoffschlüssig angeordnet gehalten werden kann.

Eine bevorzugte Weiterbildung der vorliegenden Erfindung sieht vor, dass der innere Schenkel den mindestens einen Finger umfasst.

Gemäß einer bevorzugten Weiterbildung sind mindestens zwei, vorzugsweise drei oder mehr, freistehende Finger vorgesehen. Die freistehenden elastisch auslenkbaren Finger können über den Umfang um die zweite Achse herum verteilt und zueinander beabstandet angeordnet sein und speichen- oder kammartig in die Führungsöffnung ragen. Ferner können entlang der zweiten Achse mindestens zwei elastische Finger angeordnet sein. Durch die Anzahl der elastischen Finger kann ferner die Haltekraft für die Schutzhülse ausgelegt werden.

Eine bevorzugte Weiterbildung der vorliegenden Erfindung sieht vor, dass die freistehenden und elastischen Finger umfangssymmetrisch zu der zweiten Achse angeordnet sind. Insbesondere die umfangssymmetrische Anordnung der freistehenden Finger ermöglicht eine Zentrierung der Schutzhülse in der Führungsöffnung.

Eine Weiterbildung der vorliegenden Erfindung sieht vor, dass der mindestens eine elastische auslenkbare Finger an einem freien Ende einen Kontaktbereich aufweist. Der Kontaktbereich kann konfiguriert sein, eine möglichst große Haltekraft und eine möglichst große Haftreibung zu erzeugen. Beispielsweise kann der Kontaktbereich aus einem entsprechenden Werkstoff ausgebildet sein und/oder eine entsprechend ausgestattete Oberfläche aufweisen, die einen möglichst großen Reibungskoeffizienten aufweist.

Nach Maßgabe einer Weiterbildung der vorliegenden Erfindung kann der Kontaktbereich gegenüber einer Orientierung des wenigstens einen Fingers abgewinkelt sein, wobei der Kontaktbereich konfiguriert ist, auf der Schutzhülse flächig anzulegen.

Nachfolgend werden unter Bezugnahme auf die begleitenden Zeichnungen ein Ausführungsbeispiel der erfindungsgemäßen Zielvorrichtung sowie Weiterbildungen der Zielvorrichtung im Detail beschrieben. Es zeigen:
- Figur 1a-1c: eine perspektivische Darstellung einer erfindungsgemäßen Zielvorrichtung mit einem Haltemittel, welches eine Prothese in einer ersten Achse positioniert, und einer Schutzhülse, die in einer Führungsöffnung durch Arretierungsmittel in einer ersten Achse gehalten ist,
- Figuren 2a-2c: unterschiedliche Ansichten des Arretierungsmittels gemäß Figur 1,
- Figuren 3a-3c: unterschiedliche Ansichten einer zweiten Ausgestaltung des Arretierungsmittels gemäß Figur 1,
- Figuren 4a-4c: unterschiedliche Ansichten einer dritten Ausgestaltung des Arretierungsmittels gemäß Figur 1,
- Figuren 5a-5c: unterschiedliche Ansichten einer vierten Ausgestaltung des Arretierungsmittels gemäß Figur 1,
- Figuren 6a-6d: unterschiedliche Ansichten einer fünften Ausgestaltung des Arretierungsmittels gemäß Figur 1, und
- Figur 7: eine sechste Ausgestaltung des Arretierungsmittels gemäß Figur 1.

Nachfolgend werden gleiche oder funktional gleiche Bauteile in einem Ausführungsbeispiel mit gleichen Bezugszeichen gekennzeichnet. Der Übersichtlichkeit halber sind in den einzelnen Figuren nicht alle gleichen oder funktional gleichen Teile mit einer Bezugsziffer versehen.

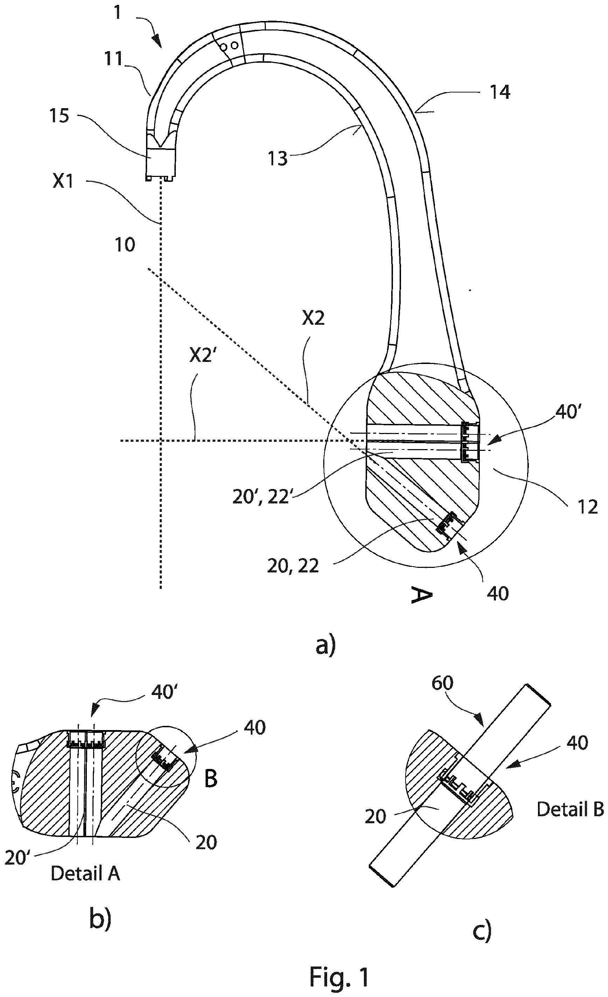

Figur 1a zeigt eine Darstellung einer Zielvorrichtung 1 gemäß einer bevorzugten Ausführungsform der Erfindung.

Die Zielvorrichtung 1 ist bügelförmig ausgebildet und weist einen ersten Endbereich 11 und einen zweiten Endbereich 12 auf und kann einen ein- oder mehrteiligen als Ganzes bezeichneten Hauptkörper 10 umfassen. Ferner weist die Zielvorrichtung 1 eine proximale Seite 13 und eine distale Seite 14 auf, wobei die proximale Seite 13 auf einem konkaven Bereich der bügelförmigen Zielvorrichtung 1 liegt und die distale Seite 14 auf einem zum konkaven Bereich gegenüberliegenden konvexen Bereich.

In dem ersten Endbereich 11 ist ein Haltemittel 15 zum Positionieren einer nicht dargestellten Prothese, insbesondere eines Marknagels, vorgesehen, wobei die Prothese entlang einer ersten Achse X1 orientiert gehalten werden kann. Die Haltemittel 15 können eine lösbare Verbindung mit der Prothese herstellen.

In dem zweiten Endbereich 12 ist mindestens eine mindestems eine Führungsöffnung 22 bildende Aufnahme 20 ausgebildet, wobei die Aufnahme 20 die Zielvorrichtung 1 bzw. den Hauptkörper 10 vollständig durchbricht und die Aufnahme 20 die proximale Seite 13 mit der distalen Seite 14 verbindet. Die Aufnahme 20 ist in einer zweiten Achse X2 orientiert, wobei, wie in dem Ausführungsbeispiel gemäß Figur 1 dargestellt ist, mehrere Aufnahmen 20 vorgesehen sind. Der Übersichtlichkeit sind in den begleitenden Figuren die weitere Aufnahme 20, die weitere Führungsöffnung 22 und die weitere zweite Achse X2 mit einem "'" gekennzeichnet.

Die erste Achse X1 und die zweite Achse X2 weisen einen gemeinsamen Schnittpunkt auf, wobei bevorzugt der Schnittwinkel auf der der Zielvorrichtung 1 zugewandten Seite näherungsweise zwischen 45° und 135° liegen sollte.

In die Aufnahme 20 bzw. in die Führungsöffnung 22 kann, wie unter anderem in den Figuren 1C, 2A, 3A, 4A und 5A gezeigt ist, eine Schutzhülse 60 eingesetzt bzw. durchgeführt werden, wobei die Schutzhülse 60 bevorzugt leichtgängig in die Aufnahme 20 und die Führungsöffnung 22 entlang der zweiten Achse X2 eingesetzt werden kann. Hierzu korrespondieren die Formen der Schutzhülse 60 und der Führungsöffnung 22 zueinander.

In die Aufnahme 20' bzw. in die Führungsöffnung 22' können in einer nicht dargestellten Weise auch zwei Schutzhülsen 60 in analoger Weise eingesetzt bzw. durchgeführt werden, oder eine Schutzhülse in unterschiedlichen Positionierungen.

Die Aufnahme 20 und/oder die Führungsöffnung 22 können einen kreisförmigen oder achtförmigen Querschnitt aufweisen, wobei die Führungsöffnung 22 ferner die Ausrichtung der Schutzhülse 60 in der zweiten Achse X2 vorgibt. Die Führungsöffnung 22 weist einen Durchmesser D22 auf und die Schutzhülse 60 einen Durchmesser D60, wobei der Durchmesser D60 bevorzugt zur Bildung einer Spielpassung geringfügig kleiner ist als der Durchmesser D22.

Ferner umfasst die Zielvorrichtung 1 ein Arretierungsmittel 40, welches konfiguriert ist, die Schutzhülse 60 in der Aufnahme 20 festzulegen um ein ungewolltes oder unbeabsichtigtes Herausfallen zu vermeiden.

Das Arretierungsmittel 40 gemäß den Figuren 1 - 7 umfasst wenigstens einen elastischen Finger 50, der in Bezug auf die zweite Achse X2 axial und/oder radial elastisch ausgelenkt werden kann und in Wirkkontakt mit der mindestens einen Schutzhülse 60 kommen kann. Der wenigstens eine elastische auslenkbare Finger 50 ragt frei beweglich in die Führungsöffnung 22 und kann beim Einsetzen der Schutzhülse 60 in die Führungsöffnung 22 eine elastische Ausweichbewegung vornehmen und anschließend mit einer Haltekraft gegen die Schutzhülse 60 drücken, um diese in der Führungsöffnung 22 in einer gewünschten Position zu fixieren.

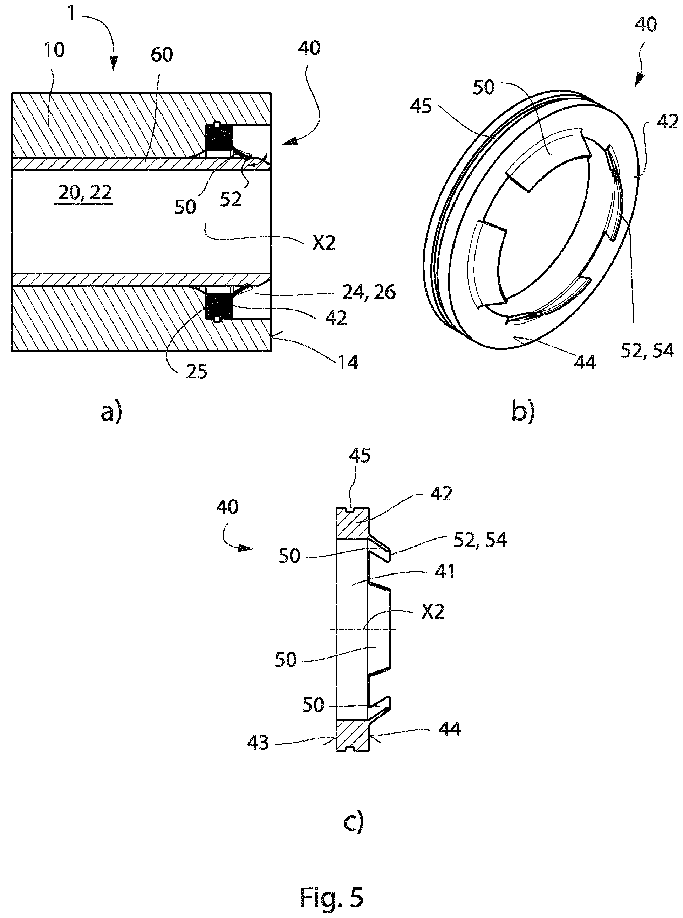

Der wenigstens eine elastische Finger 50 ragt in Bezug auf die zweite Achse X2 radial oder radial und axial in die Führungsöffnung 22. Ein freies Ende 52 des jeweiligen Fingers 50 befindet sich im nicht ausgelenkten Zustand des elastischen Fingers 50 innerhalb der Führungsöffnung 22. An dieser Stelle wird angemerkt, dass in den begleitenden Figuren die Finger 50 aus Gründen der vereinfachten Darstellung im nicht ausgelenkten Zustand dargestellt sind. Lediglich in Figur 5a wird eine Auslenkbewegung mittels gestrichelten Linien sowie anhand eines Doppelpfeils angedeutet.

Das freie Ende 52 kann einen Kontaktbereich 54 aufweisen, der bevorzugt erhöhte Haftreibungseigenschaften aufweist.

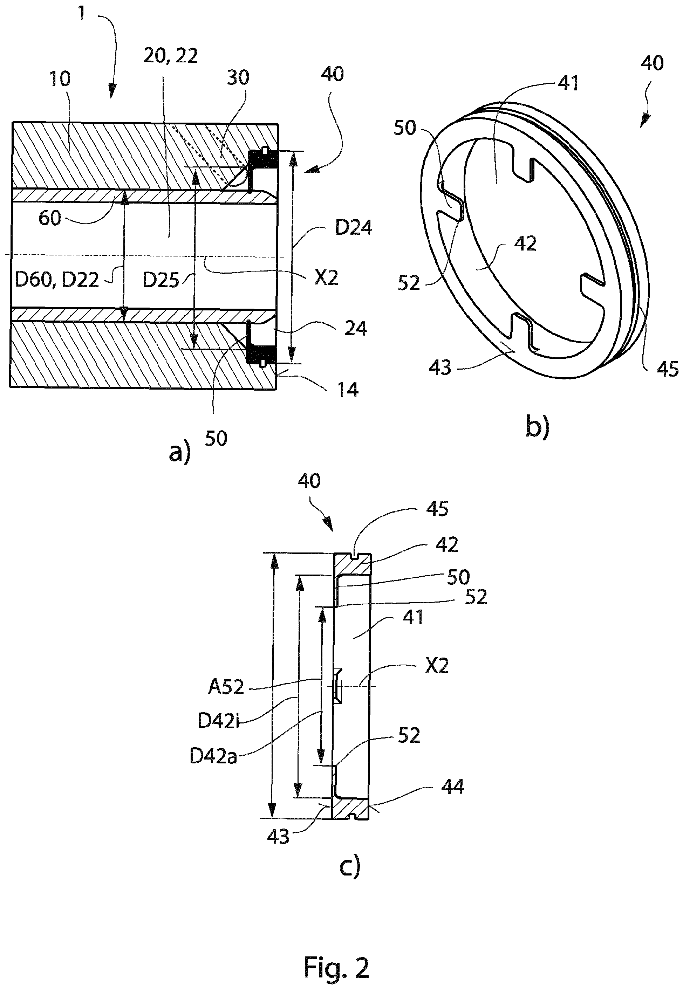

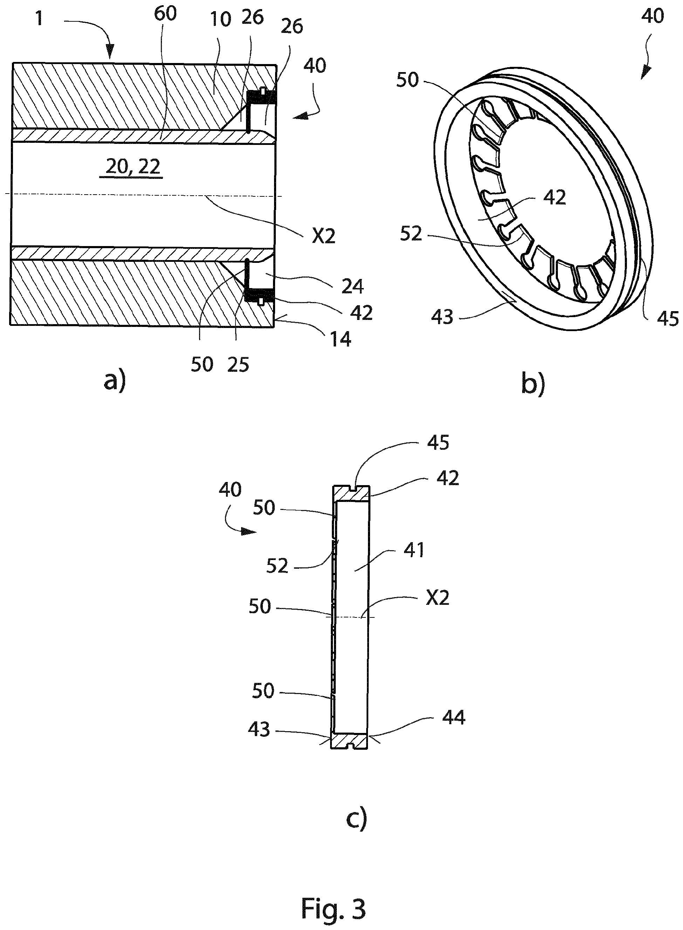

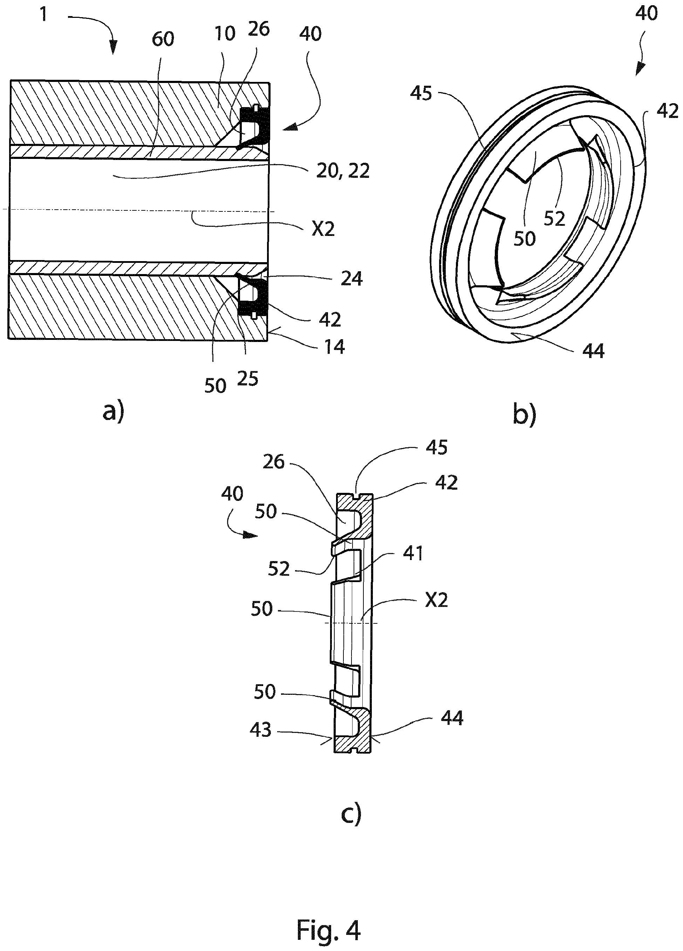

Beispielsweise ragen die elastischen auslenkbaren Finger 50 gemäß den Ausführungsbeispielen nach den Figuren 2 und 3 radial in die Führungsöffnung 22, währenddessen die elastischen Finger 50 gemäß den Ausführungsbeispielen nach den Figuren 4 und 5 in einem Winkel von ca. 45° geneigt zu der zweiten Achse X2 orientiert sind, und demnach radial und axial orientiert in die Führungsöffnung 22 ragen.

Das Arretierungsmittel 40 kann, wie in den dargestellten Ausführungsbeispielen gezeigt ist, eine Buchse 42 umfassen, die in die Aufnahme 20 eingesetzt werden kann. Von der Buchse 42 stehen die elastischen Finger 50 freistehend in Bezug auf die zweite Achse X2 in der radialen Richtung oder in der radialen und axialen Richtung in Richtung der zweiten Achse X2 ab.

Die Aufnahme 20 kann auf der proximalen Seite 13 und/oder auf der distalen Seite 14 eine Aufweitung 24 aufweisen, die konfiguriert ist, die Buchse 42 aufzunehmen. Die Aufweitung 24 weist einen Durchmesser D24 auf, wobei der Durchmesser D24 größer ist als der Durchmesser D22.

Die Buchse 42 weist einen Außendurchmesser D42a und die Durchbrechung der Buchse 42 einen Innendurchmessen D42i auf. Der Außendurchmesser D42a korrespondiert mit dem Durchmesser D24 der Aufweitung 24 und der Innendurchmesser D42i ist größer als der Durchmesser D60 der Schutzhülse 60.

Die Buchse 42 kann in der Aufnahme 20 durch Verbindungsmittel kraftschlüssig, formschlüssig und/oder stoffschlüssig angeordnet gehalten werden. Beispielsweise kann die stoffschlüssige Anordnung der Buchse 42 in der Aufnahme 20 vorteilhaft sein, um das Eindringen von Verunreinigungen in der Schnittstelle zwischen der Buchse 42 und dem Hauptkörper der Zielvorrichtung 1 zu vermeiden. Hierzu kann die Buchse 42 einen Einstich 45 aufweisen, in den ein Dichtmittel und/oder ein Klebstoff eingebracht werden kann.

Die Aufweitung 24 kann ferner einen als Absatz ausgebildeten Anschlag 25 umfassen, durch den die Position der Buchse 42 in der Aufnahme 20 vorgegeben sein kann. Eine der Stirnseiten 43, 44 der Buchse 42 kann an dem Anschlag 25 in Anlage kommen.

Unter Bezugnahme auf die Figur 2a ist ersichtlich, dass ferner in der Aufnahme 20 ein Ringraum 26 benachbart zu der Aufweitung 24 ausgebildet ist, in den der wenigstens eine elastische Finger 50 eintauchen kann.

Der Ringraum 26 kann durch einen konischen Abschnitt gebildet werden, der die Aufweitung 24 bzw. den Anschlag 25 mit der Führungsöffnung 22 verbindet. Der größte Durchmesser D25 des konischen Ringraums 26 oder eines zylindrischen Ringsraums 26 sollte größer sein als der Innendurchmesser D42i, um eine unbehinderte Auslenkbewegung des elastischen Fingers 50 zu ermöglichen.

In den Figuren 2-7 sind unterschiedliche Ausgestaltungen des zuvor allgemein beschriebenen Arretierungsmittels 40 dargestellt, wobei die nachfolgend detailliert beschriebenen Arretierungsmittel 40 die oben beschriebenen Eigenschaften gemeinsam haben.

Das Arretierungsmittel 40 gemäß der Figur 2 weist mehrere um die zweite Achse X2 umfangssymmetrisch angeordnete elastische Finger 50 auf, die radial in Richtung der zweiten Achse X2 in eine Durchbrechung 41 der Buchse 42 bzw. in die Führungsöffnung 22 ragen. Der Abstand A52 zwischen zwei diametral angeordneten freien Enden 52 ist kleiner als der Durchmesser D60.

Ferner ist in der Figur 2 angedeutet, dass ein Spülkanal 30 vorgesehen werden kann, der in den Ringraum 26 mündet. Der Spülkanal 30 ist konfiguriert, ein Reinigungsmittel in den Ringraum 26 zu leiten, um beispielsweise die Zielvorrichtung 1 nach der Verwendung zu reinigen bzw. zu sterilisieren. Der Spülkanal 30 kann bevorzugt in einer zu der zweiten Achse X2 tangential orientierten Richtung in den Ringraum 26 münden, wodurch der Ringraum 26 bei der Reinigung effektiv durchspült werden kann.

Das Arretierungsmittel 40 gemäß Figur 3 ist analog zu dem Arretierungsmittel 40 gemäß Figur 2 ausgebildet, wobei eine Mehrzahl von elastischen Fingern 50 engmaschig vorgesehen ist. In der Umfangsrichtung ist der Abstand zwischen den elastischen Fingern 50 kleiner, als die Breite der elastischen Finger 50. Ein Freiraum zwischen den elastischen Fingern 50 weist auf der von dem freien Ende 52 abgewandten Seite eine Entlastungsaufbohrung auf. Die Entlastungsaufbohrung kann die Kerbwirkung des kerbenförmigen Freiraums reduzieren.

Figur 4 zeigt ein im Querschnitt U-förmiges Arretierungsmittel 40. Das Arretierungsmittel 40 ist vergleichbar zu den bereits vorgestellten Arretierungsmitteln 40 gemäß den Figuren 2 und 3 ausgestaltet. Allerdings ist hier die Buchse 42 im Querschnitt U-förmig ausgebildet, und weist einen inneren Schenkel, einen äußeren Schenkel und einen mittleren Schenkel auf. Der mittlere Schenkel verbindet den inneren Schenkel und den äußeren Schenkel, wobei die Schenkel den Ringraum 26 zumindest bereichsweise umschließen. Der innere Schenkel bildet die elastischen Finger 50 aus, wodurch eine besonders kompakte Bauweise des Arretierungsmittels 40 bewerkstelligt werden kann.

Eine weitere Ausgestaltung des Arretierungsmittels 40 ist Figur 5 zu entnehmen, wobei die elastischen Finger von einer Stirnseite 43, 44 der ringförmigen Buchse 42 abstehen.

Die das Arretierungsmittel 40 bildende Buchse 42 kann in einer beliebigen Orientierung in die Aufnahme 20 eingesetzt werden, wobei die elastisch auslenkbaren Finger 50 in Bezug auf die zweite Achse X2 in beide Richtungen weisen können.

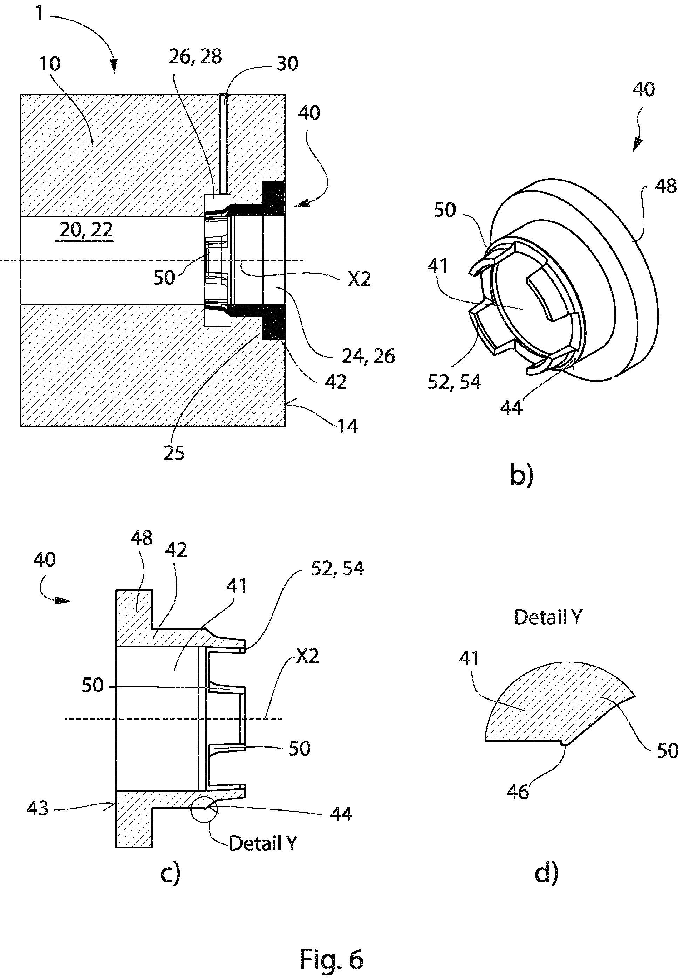

Eine fünfte Ausgestaltung des Arrretierungsmittels 40 und eine Weiterbildung des Hauptkörpers 10 wird in Figur 6 gezeigt. Der Hauptkörper weist in der Aufweitung 24 eine Hinterschneidung 28 auf, die von der distalen und/oder proximalen Seite 14 beabstandet ist. Die Hinterschneidung 28 kann den Ringraum 26 ausbilden. Die auslenkbaren Finger 50 stehen freibeweglich in den Ringraum 26 und können so eine ungehinderte Ausweichbewegung vornehmen.

Wie Figur 6a zu entnehmen ist, kann in vergleichbarer Weise, wie bereits beschrieben, ein Spülkanal 30 vorgesehen werden, der in den Ringraum 26 mündet.

Das Arretierungsmittel 40 gemäß den Figuren 6a - 6d ist vergleichbar zu den bereits vorgestellten Arretierungsmitteln 40 gemäß den Figuren 4 und 5 ausgestaltet. Allerdings ist hier die Buchse 42 in der zweiten Achse X2 länglicher ausgebildet und der elastische Finger 50 steht von einer Stirnseite 43, 44 der Buchse 42 ab. Die gegenüberliegende Stirnseite 43 oder 44 weist einen Flanschabschnitt 48 auf, der mit dem Anschlag 25 zusammenwirken kann, um die Lage des Arretierungsmittels 40 in der zweiten Achse X2 in der Aufnahme 20 festzulegen.

Aus der Detaildarstellung gemäß Figur 6d kann entnommen werden, dass das Verbindungsmittel des Arretierungsmittels 40 mindestens einen Vorsprung 46 umfassen kann, um das Arretierungsmittel 40 in der Aufnahme 20 kraftschlüssig zu halten. Der Vorsprung 46 kann als umlaufender Flansch ausgebildet sein und ist konfiguriert, das Arretierungsmittel 40 durch eine Übermaßpassung in der Aufnahme 20 geklemmt zu halten. Auch können über den Umfang verteilt mehrere zahnförmige Vorsprünge 46 vorgesehen werden.

Der Vorsprung 46 ist bevorzugt benachbart zu den auslenkbaren Fingern 50 angeordnet. In dem dargestellten Ausführungsbeispiel ist der Vorsprung 46 entlang der zweiten Achse X2 in dem Bereich ausgebildet, von dem der Finger 50 absteht, oder genauer gesagt, benachbart zu der Stirnseite 43, 44 angeordnet, von welcher der wenigstens eine Finger 50 absteht.

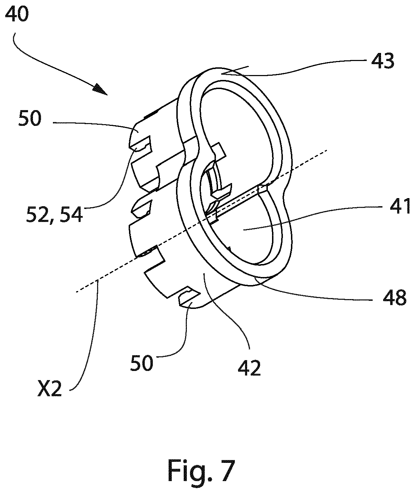

Figur 7 zeigt eine sechste bespielhafte Ausgestaltung des Arretierungsmittels 40, gemäß Figur 1b welches dort dem Bezugszeichen 40' versehen ist. Das Arretierungsmittel 40 gemäß Figur 7 weist im Unterschied zu dem in Figur 6 dargestellten Arretierungsmittel 40 eine im Querschnitt achtförmige Durchbrechung 41 auf, die es ermöglichen soll, dass eine (nicht dargestellte) Schutzhülse 60 in zwei Positionierungen parallel und beabstandet zu der zweiten Achse X2' eingesetzt und gehalten werden kann. Die im Querschnitt achtförmige Durchbrechung 41 kann derart ausgebildet sein, dass die zwei die zwei die Querschnittsfläche umrahmenden Kreise an einem oder zwei Schnittpunkten schneiden. Auch können die umrahmenden Kreise zueinander beabstandet angeordnet sein. In einen solchen Fall können zweitgleich zwei Schutzhülsen 60 durch das Arretierungsmittel 40 gehalten werden.

### Bezugszeichenliste

- 1: Zielvorrichtung
- 5: Knochenbefestigungselement
- 8: Prothese
- 10: Hauptkörper
- 11: erster Endbereich
- 12: zweiter Endbereich
- 13: proximale Seite
- 14: distale Seite
- 15: Haltemittel
- 20: Aufnahme
- 22: Führungsöffnung
- 24: Aufweitung
- 25: Anschlag
- 26: Ringraum
- 30: Spülkanal
- 40: Arretierungsmittel
- 41: Durchbrechung
- 42: Buchse
- 43: erste Stirnseite
- 44: zweite Stirnseite
- 45: Einstich
- 46: Vorsprung
- 48: Flansch
- 50: Finger
- 52: freies Ende von 50
- 54: Kontaktbereich
- 60: Schutzhülse

- X1: erste Achse
- X2: zweite Achse

## Patentansprüche

1. Zielvorrichtung (1) zum Ausbilden einer Befestigung einer Prothese (8) mit einem Knochenbefestigungselement (5) an einem Knochen, aufweisend
- Haltemittel (15) zum Positionieren der Prothese (8) in einer ersten Achse (X1),
- eine eine Führungsöffnung (22) bildende Aufnahme (20), die entlang einer zweiten Achse (X2) orientiert ist und die Zielvorrichtung durchbricht und ausgebildet ist, eine Schutzhülse aufzunehmen, und
- ein Arretierungsmittel (40), das wenigstens einen elastisch auslenkbaren Finger (50) umfasst, der in Bezug auf die zweite Achse (X2) axial und/oder radial ausgelenkt werden kann und eingerichtet ist, die Schutzhülse entlang der zweiten Achse (X2) festzulegen,
- wobei sich die erste Achse (X1) und die zweite Achse (X2) kreuzen,
- wobei der wenigstens eine Finger (50) in die Führungsöffnung (22) ragt,
**dadurch gekennzeichnet,**
**dass** das Arretierungsmittel (40) eine Buchse (42) umfasst.

2. Zielvorrichtung (1) nach Anspruch 1,
**dadurch gekennzeichnet, dass** die Buchse (42) in der Aufnahme (20) anordnenbar ist.

3. Zielvorrichtung (1) nach einem der vorgenannten Ansprüche,
**dadurch gekennzeichnet, dass** ein Ringraum (26) in der Aufnahme (20) ausgebildet ist, und dass der mindestens eine Finger (50) in den Ringraum (26) freibeweglich ragen kann.

4. Zielvorrichtung (1) nach Anspruch 3,
**dadurch gekennzeichnet, dass** in den Ringraum (26) ein Spülkanal (30) mündet.

5. Zielvorrichtung (1) nach Anspruch **4,**
**dadurch gekennzeichnet, dass** der Spülkanal (30) in einer zu der zweiten Achse (X2) tangential orientierten Richtung in der Aufnahme (30) mündet.

6. Zielvorrichtung (1) nach einem der vorgenannten Ansprüche,
**dadurch gekennzeichnet, dass** das Arretierungsmittel (40) im Querschnitt U-förmig ist.

7. Zielvorrichtung (1) nach einem der vorgenannten Ansprüche,
**dadurch gekennzeichnet, dass** Verbindungsmittel vorgesehen sind, durch die das Arretierungsmittel (40) in der Aufnahme (20) kraftschlüssig, formschlüssig und/oder stoffschlüssig angeordnet gehalten werden kann.

8. Zielvorrichtung (1) nach einem der vorgenannten Ansprüche,
**dadurch gekennzeichnet, dass** das Arretierungsmittel (40) aus einem Kunststoff ist.

9. Zielvorrichtung (1) nach einem der vorgenannten Ansprüche,
**dadurch gekennzeichnet, dass** mehrere freistehende Finger (50) vorgesehen sind.

10. Zielvorrichtung (1) nach einem der vorgenannten Ansprüche,
**dadurch gekennzeichnet, dass** die freistehenden Finger (50) umfangssymmetrisch zu der zweiten Achse (X2) angeordnet sind.

11. Zielvorrichtung (1) nach einem der vorgenannten Ansprüche,
**dadurch gekennzeichnet, dass** der mindestens eine Finger (50) an einem freien Ende (52) einen Kontaktbereich (54) aufweist.

12. Zielvorrichtung (1) nach Anspruch 11,
**dadurch gekennzeichnet,dass** der Kontaktbereich (54) des mindestens einen Fingers (50) derart abgewinkelt ist, um flächig an der Schutzhülse anzuliegen.

## Claims

1. Aiming device (1) for forming a fastening of a prosthesis (8) to a bone fastening element (5) on a bone, comprising:
- holding means (15) for positioning the prosthesis (8) in a first axis (X1),
- a receptacle (20) that forms a guide opening (22), which is oriented along a second axis (X2) and penetrates the aiming device and is configured for receiving a protective sleeve, and
- a locking means (40) which comprises at least one resiliently deflectable finger (50) which dan be deflected axially and/or radially with respect to the second axis (X2) and is configured for fixing the protective sleeve along the second axis (X2),
- wherein the first axis (X1) and the second axis (X2) intersect,
- wherein the at least one finger (50) protrudes into the guide opening (22),
**characterised in that**
the locking means (40) comprises a bush (42).

2. Aiming device (1) according to claim 1,
**characterised in that**
the bush (42) can be arranged in the receptacle (20).

3. Aiming device according to either of the preceding claims,
**characterised in that**
an annular space (26) is formed in the receptacle (20), and **in that** the at least one finger (50) can protrude into the annular space (26) in a freely movable manner.

4. Aiming device (1) according to claim 3,
**characterised in that**
a rinsing channel (30) leads into the annular space (26).

5. Aiming device (1) according to claim 4,
**characterised in that**
the rinsing channel (30) leads into the receptacle (30) in a direction oriented tangentially to the second axis (X2).

6. Aiming device (1) according to any of the preceding claims,
**characterised in that**
the locking means (40) is U-shaped in cross-section.

7. Aiming device (1) according to any of the preceding claims,
**characterised in that**
connecting means are provided, by which the locking means (40) can be held in the receptacle (20) so as to be arranged in a force-fitting, form-fitting and/or integrally locking manner.

8. Aiming device (1) according to any of the preceding claims,
**characterised in that**
the locking means (40) is made of a plastics material.

9. Aiming device (1) according to any of the preceding claims,
**characterised in that**
a plurality of free-standing fingers (50) is provided.

10. Aiming device (1) according to any of the preceding claims,
**characterised in that**
the free-standing fingers (50) are arranged in a peripherally symmetrical manner with respect to the second axis (X2).

11. Aiming device (1) according to any of the preceding claims,
**characterised in that**
the at least one finger (50) comprises a contact region (54) at a free end (52).

12. Aiming device (1) according to claim 11,
**characterised in that**
the contact region (54) of the at least one finger (50) is angled so as to lie in a planar manner on the protective sleeve.

## Revendications

1. Dispositif (1) de visée pour la constitution d'une fixation d'une prothèse (8) à un os par un élément (5) de fixation à l'os, comportant
- un moyen (15) de maintien pour la mise en position de la prothèse (8) dans un premier axe (X1),
- un logement (20), qui forme une ouverture (22) de guidage, qui est orienté suivant un deuxième axe (X2) et qui perce le dispositif de visée, et est constitué pour recevoir un manchon de protection, et
- un moyen (40) d'arrêt, qui comprend au moins un doigt (50) pouvant être dévié élastiquement, qui peut être dévié axialement et/ou radialement par rapport au deuxième axe (X2) et qui est agencé pour fixer le manchon de protection le long du deuxième axe (X2),
- dans lequel le premier axe (X1) et le deuxième axe (X2) se croisent,
- dans lequel le au moins un doigt (50) pénètre dans l'ouverture (22) de guidage,
**caractérisé**
**en ce que** le moyen (40) d'arrêt comprend une douille (42).

2. Dispositif (1) de visée suivant la revendication 1,
**caractérisé en ce que**
la douille (42) peut être montée dans le logement (20).

3. Dispositif (1) de visée suivant l'une des revendications précédentes,
**caractérisé en ce qu'**
un espace (26) annulaire est constitué dans le logement (20) et **en ce que** le au moins un doigt (50) peut pénétrer avec la possibilité de se déplacer librement dans l'espace (26) annulaire.

4. Dispositif (1) de visée suivant la revendication 3,
**caractérisé en ce qu'**
un conduit (30) de lavage débouche dans l'espace (26) annulaire.

5. Dispositif (1) de visée suivant la revendication 4,
**caractérisé en ce que**
le conduit (30) de lavage débouche dans le logement (30) dans une direction orientée tangentiellement au deuxième axe (X2).

6. Dispositif (1) de visée suivant l'une des revendications précédentes,
**caractérisé en ce que**
le moyen (40) d'arrêt est en forme de U en section transversale.

7. Dispositif (1) de visée suivant l'une des revendications précédentes
**caractérisé en ce qu'**
il est prévu des moyens d'assemblage, par lesquels le moyen (40) d'arrêt peut être maintenu à coopération de force, à complémentarité de forme et/ou à coopération de matière dans le logement (20).

8. Dispositif (1) de visée suivant l'une des revendications précédentes,
**caractérisé en ce que**
le moyen (40) d'arrêt est en une matière plastique.

9. Dispositif (1) de visée suivant l'une des revendications précédentes,
**caractérisé en ce qu'**
il est prévu plusieurs doigts (50) indépendants.

10. Dispositif (1) de visée suivant l'une des revendications précédentes,
**caractérisé en ce que**
les doigts (50) indépendants sont disposés à symétrie de pourtour par rapport au deuxième axe (X2).

11. Dispositif (1) de visée suivant l'une des revendications précédentes,
**caractérisé en ce que**
le au moins un doigt (50) a une partie (54) de contact à une extrémité (52) libre.

12. Dispositif (1) suivant la revendication 11,
**caractérisé en ce que**
la partie (54) de contact du au moins un doigt (50) est coudée, de manière à s'appliquer par une surface au manchon de protection.
